# EUROPEAN PATENT SPECIFICATION

(11) **EP 4 022 623 B1**
(45) Date of publication and mention of the grant of the patent: **01.11.2023**
(21) Application number: 20751143.7
(22) Date of filing: 05.08.2020
(51) Int. Cl.: G16B 25/10

(54) **METHOD AND KIT TO PREDICT CELL DEATH IN RESPONSE TO BIOTIC AND/OR ABIOTIC STIMULI**
VERFAHREN UND KIT ZUR VORHERSAGE DES ZELLTODS IN REAKTION AUF BIOTISCHE UND/ODER ABIOTISCHE STIMULI
PROCÉDÉ ET KIT POUR PRÉDIRE LA MORT CELLULAIRE EN RÉPONSE À DES STIMULI BIOTIQUES ET/OU ABIOTIQUES

(30) Priority: 26.08.2019 IT 201900012624
(43) Date of publication of application: 06.07.2022
(73) Proprietor: Stazione Zoologica Anton Dohrn, 80121 Napoli (NA) (IT)
(72) Inventor: IANORA, Adrianna, 80121 Napoli (NA) (IT); SANSONE, Clementina, 80121 Napoli (NA) (IT); CHIUSANO, Maria Luisa, 80121 Napoli (NA) (IT); ROMANO, Giovanna, 80121 Napoli (NA) (IT); GALASSO, Christian, 80121 Napoli (NA) (IT); TANGHERLINI, Michael, 80121 Napoli (NA) (IT); BRUNET, Christophe, 80121 Napoli (NA) (IT)
(74) Representative: Currado, Luisa
(86) International application number: PCT/EP2020/071981
(87) International publication number: WO 2021/037503

(56) References cited:
- WO-A1-2018/204764
- AFTAB ALAM ET AL: "Identification and Classification of Differentially Expressed Genes and Network Meta-Analysis Reveals Potential Molecular Signatures Associated With Tuberculosis", FRONTIERS IN GENETICS, vol. 10, 10 September 2019 (2019-09-10), XP055688459, DOI: 10.3389/fgene.2019.00932
- FLORIAN UHLITZ ET AL: "signal duration", MOLECULAR SYSTEMS BIOLOGY, vol. 13, no. 5, 1 May 2017 (2017-05-01), page 928, XP055688447, GB ISSN: 1744-4292, DOI: 10.15252/msb.20177554

## Description

### Background of the invention

The present invention refers to the field of biotechnology and diagnostic since it describes an in vitro and ex vivo method for the prediction of cell fate and/or death in response to stimuli and/or stresses.

### State of the Art

Cellular homeostasis is an important mechanism useful for tissue integrity maintenance in multicellular organisms. For this purpose, in all organisms, apoptosis is a highly conserved mechanism that has evolved to maintain cell numbers and cellular positioning within tissues. Cellular functions are regulated by highly complex signalling processes interconnected by thousands of different genes that control cellular growth, migration, metabolism, differentiation and cell death. In mammals, there are two major apoptotic pathways, the extrinsic pathway (death receptor pathway) or the intrinsic pathway (the mitochondrial pathway) within a cell. The extrinsic pathway is activated by apoptotic stimuli comprising extrinsic signals such as the binding of death inducing ligands to cell surface receptors. In other cases, apoptosis is initiated following intrinsic signals including DNA damage induced by irradiation or chemicals, growth factor deprivation or oxidative stress. In general, intrinsic signals initiate apoptosis via the involvement of the mitochondria. Death-related processes can be classified as inflammation-dependent pathways, e.g. extrinsic apoptosis, necroptosis, autophagy, pyroptosis, pyronecrosis, patraptosis and entosis; and inflammation-independent or homeostatic pathways, e.g. intrinsic apoptosis, necrosis, mitophagy, ribophagy and NFkb1 signalling. Each of these involves specific gene responses. Gene expression analysis can help the understanding of specific pathway activation. Several experimental methodologies are currently available for gene expression analysis focusing on single, such as PCR-related, or multiple, such as microarray and transcriptome sequencing (Kerr JF, Wyllie AH, Currie AR. Apoptosis: A basic biological phenomenon with wide-ranging implications in tissue kinetics. Br J Cancer 1972; 26:239-57; Green DR, Knight RA, Melino G, Finazzi-Agro A, Orrenius S. Ten years of publication in cell death. Cell Death Differ 2004; 11:2-3; Stephan Knapp. Cell signalling pathways. Chapter 3 in: David J. Kerr, Daniel G. Haller, Cornelis J. H. van de Velde, Michael Baumann. Oxford University Press, 28 gen 2016 - 832 pp).

Previously, a dynamic BH3 Profiling technique to measure early changes in pro-apoptotic signalling at the mitochondrion level was developed, defined as priming, induced by chemotherapeutic agents in cancer cells, where the early administration of a drug induces a death-signalling pathway measured by a dynamic BH3 Profiling that predicts the chemotherapeutic response across many cancer types (Joan Montero, Kristopher A. Sarosiek, Joseph D. De Angelo, Ophelia Maertens, Jeremy Ryan, Dalla Ercan, Huiying Piao, Neil S. Horowitz, Ross S. Berkowitz, Ursula Matulonis, Pasi A. Janne, Philip C. Amrein, Karen Cichowski, Ronny Drapkin, and Anthony Letai. Drug-Induced Death Signaling Strategy Rapidly Predicts Cancer Response to Chemotherapy. Cell 160, 977-February 26, 20152015 Elsevier Inc.)

The expression of apoptotic genes in A549 human lung adenocarcinoma cells, COLO 205 colon adenocarcinoma metastatic cells and BEAS-2B lung/brunch normal epithelial cell line has been investigated (Sansone C. et al, 2014, PLoS ONE, Diatom-Derived Polyunsaturated Aldehydes Activate Cell Death in Human Cancer Cell Lines but Not Normal Cells, 9(7): e101220. doi:10.1371/journal.pone.0101220).

The expression of genes involved in oxidative stress and repairing pathways in A549 cells has been disclosed (Sansone C. et al, 2017, Sci Rep, The green microalga Tetraselmis suecica reduces oxidative stress and induces repairing mechanisms in human cells, 7:41215 | DOI: 10.1038/srep41215).

The genes involved in human death cell signalling pathways in 4-HBA treated A549 cells have been investigated (Sannino F. et al., 2018, Nature, Pseudoalteromonas haloplanktis TAC125 produces 4-hydroxybenzoic acid that induces pyroptosis in human A459 lung adenocarcinoma cells (2018) 8:1190).

It is known that in A549 lung cancer cells and HT29 colorectal cancer cells are activated by two different cell death pathways with the involvement of the tumor necrosis factor (TNF) cell signalling cascade and in In HT29 cells also a death signaling pathway in response to DNA damage is induced (Sansone C. et al, 2018, Marine Biotechnology, The Marine Dinoflagellate Alexandrium andersoni Induces Cell Death in Lung and Colorectal Tumor Cell Lines, https://doi.org/10.1007/s10126-018-9817-5) .

International patent application, publication n. WO2013120092 discloses methods and assays for predicting prognosis and/or response to therapies in cancer patients. In particular for predicting prognosis or response to the treatment with Bacillus Calmette-Guerin (BCG). The methods involve obtaining peripheral blood mononuclear cells (PBMC) from a subject with melanoma and culturing the PBMC with BCG prior to analysis of gene expression in the PBMC.

US patent application, publication n. 2005227352 discloses the elucidation of a signal transduction pathway controlling the expression of a gene necessary for differentiation of stem daughter cells, and the control of stem cell self-renewal and differentiation by modulating the levels of key regulatory molecules within the pathway.

US patent application, publication n. 2015141289 compositions and methods for identifying and validating modulators of cell fate such as maintenance, cell specification, cell determination, induction of stem cell fate, cell differentiation, cell dedifferentiation, and cell trans-differentiation, based on the use of reporter nucleic acid constructs, host cells comprising such constructs, and methods using such cells and constructs and high throughput screens.

International patent application, publication n. WO2007049043 discloses methods and compositions for inducing cell survival and proliferation, or cell differentiation and/or apoptosis by influencing relative flux through TNFa signalling through TNFR2 and NGF differentiation signalling pathway. Stem cells and/or neural cells are treated with activators of TNFR2 for promoting cell survival and/or for treating neurodegenerative conditions.

International patent application, publication n. WO2015163826 discloses a method of predicting response of a cancer patient to a therapy comprising determining a presence or absence of at least one mutation of ITCI-1 in a first sample isolated from a cancer patient, wherein the presence of a mutation is predictive of response of the cancer patient to a therapy selected from the group consisting of: Wnt pathway-, EGFR, Her2-, hormonal and WBP2 based therapy.

US patent application, publication n. 2015376710 discloses a method of evaluating a cancer patient comprising evaluating gene expression levels in a patient sample, calculating a predictor score using the gene expression levels and assessing the likelihood of a therapeutic outcome using the predictor score.

US patent application, publication n. 2015362479 discloses methods of predicting cell sensitivity or resistance to a therapeutic agent.

International patent application, publication n. 2009138392 discloses a method for the determination of biomarker assays and/or drug-targets for the diagnosis of cancer and its treatment and/or prognosis, specifically for prostate cancer.

International patent application n. WO 2018/204764 discloses in vitro or ex vivo methods for predicting the cell fate or death in response to stimuli by measuring differential gene expression between a group of the cells contacted with the stimulus and a group of the cells not contacted with the stimulus.

The scientific paper Aftab Alam et al., "Identification and Classification of Differentially Expressed Genes and Network Meta-Analysis Reveals Potential Molecular Signatures Associated With Tuberculosis", Frontiers in Genetics,vol. 10, 2019-11-04) discloses the classification of differentially expressed genes and the assignment of pathways to said genes.

The scientific paper Florian Uhlitz et al., "Signal duration", Molecular Systems Biology, vol. 13, no. 5, (2017-05-01), page 928, discloses a differential gene expression profiling and attributing genes to different signalling pathways such as proliferation and apoptosis.

### Technical problem

Predicting signalling pathways in cancer cells could help in the rationale design of therapeutic strategies that target the signal transduction pathways or for an early prediction of the efficacy of the strategy on a specific cellular system.

The processes known in the art, which are available to predict cell fate in relation to death or survival, present several drawbacks.

Usually they can predict one type of cell fate per experiment, because they are based on analysis of single pathways or allow the identification of one type of stress or pathological condition per time.

This limitation of single markers to predict cell fate and death lead to the obtaining of incomplete information and/or ambiguity about the associated processes that can occur when a specific signalling pathway is activated.

Moreover, the methods nowadays available take around 1-2 months to detect cell response to stimuli and/or cell death while early assessment results much more advantageous to change therapy or to test its effects.

It is clear that no comprehensive diagnostic methods are available to predict cell fate in relation to death or survival.

In view of the drawbacks of the techniques known in the art the inventors of the present invention investigated the possibility of contemporarily monitoring several pathways involved in cell reaction to stimuli.

The technical problem of the present invention is to provide a method for the detection of cell fate and death which is fast, reliable, unambiguous, user friendly, affordable and can be used with any kind of cell and any kind of stress and stimuli.

The process of the present invention provides a in vitro or ex vivo method for the prediction of cell development, fate and death in response to external or internal stimuli and stresses, which can be used for research and/or clinical purposes and to test in vitro cell cultures.

### Object of the invention

With reference to the claims, the above technical problem is solved by providing an in vitro or ex vivo method to predict the cell fate and/or death in response to stimuli and/or stresses, comprising the following steps:
a) collecting a cellular sample from biological material
b) selecting a set of known genes (A to M) involved in cell signalling pathways of the cells contained in the cellular sample from biological material of step a)
c) exposing the cellular sample from biological material of step a) to a stressor element
d) subjecting the cellular sample from biological material exposed to a stressor element as obtained in step c) to gene expression analysis to obtain genes belonging to the set of genes as selected in step b)
e) analysing the data regarding gene expression analysis as obtained in step e) by the algorithm 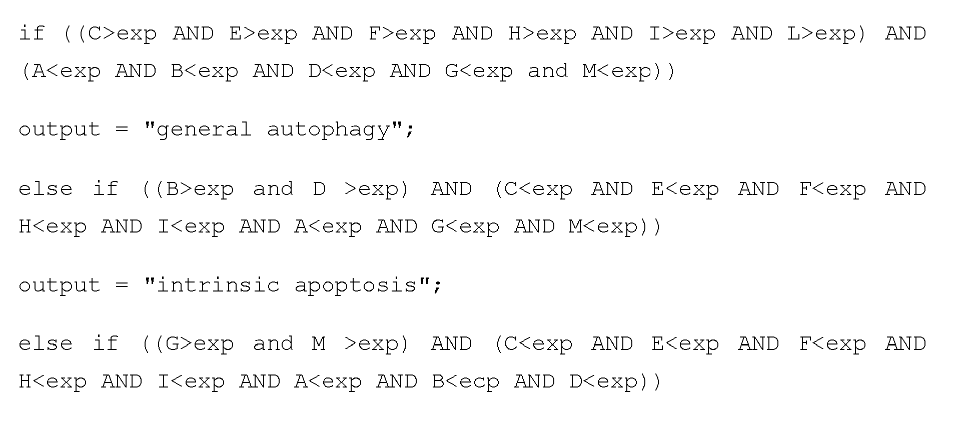 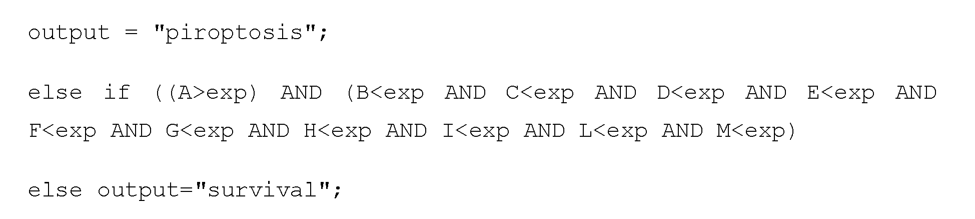
   wherein A to M are genes selected in step b)
   wherein exp is the two fold value of the expression level calculated with the equation: exp=2^((-average(Delta ct (gene expression in treated cells)))/(-average(Delta ct gene expression in untreated cells)))
   wherein Delta ct= Ct value reference gene - average of the Ct values of at least 3 housekeeping genes
   to calculate the scores of outputs: general autophagy, intrinsic apoptosis, piroptosis, survival
   wherein the output with the higher value is predictive of the cell fate
   wherein input is Ct values or gene expression data as reported from the specific trascriptome assessment
   wherein processing is validation of most probable pathways (listed classes of processes monitored)
   wherein output is processes in course; probability of process in course; unprediction.

Further features of the invention will be clear from the following detailed description with reference to the attached drawings and experimental results provided.

### Brief description of the drawings

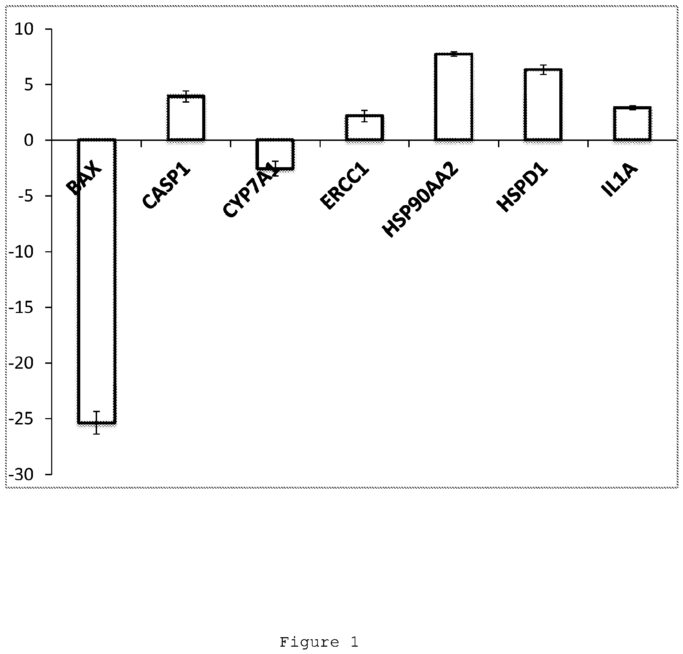

Figure 1 shows the up-regulation of key genes involved in pyroptosome formation mediating inflammatory cell death via caspase-1 activation on lung adenocarcinoma cell line treated with a pure chemical fraction isolated from the marine microalga *Cyclotella cryptica.*

### Detailed description of the invention

### Definitions

Within the meaning of the present invention cell fate means death or survival.

Within the meaning of the present invention cell death means when the cell ceases to carry out its functions due to death inducing factors which activate autophagy, intrinsic apoptosis, piroptosis, necrosis.

Within the meaning of the present invention general autophagy is an adaptive response to stress wherein unnecessary or dysfunctional cytoplasmic constituents are isolated from the rest of the cell within a double-membraned vesicle, degraded and recycled.

Within the meaning of the present invention intrinsic apoptosis means mitochondrial mediated degradation of cellular constituents by cysteine proteases caspases characterized by permeabilization of the mitochondria and release of cytochrome c into the cytoplasm, which forms multi-protein complex apoptosome and initiates activation of the caspase cascade through caspase 9.

Within the meaning of the present invention piroptosis means inflammation induced cell death mainly caused by microbial infections.

Within the meaning of the present invention necrosis means is cell death caused by external factors such as trauma or infection.

Within the meaning of the present invention cellular sample from biological material means any sample which contains cells whose genome comprises genes involved in cellular signalling leading to cell death and housekeeping genes.

The cellular sample from biological material can be tissue sample, primary cell cultures, secondary cell cultures, cell lines from collection, blood sample, preferably human. Cells and or tissues can be normal or cancerous depending on the study to be conducted. Within the meaning of the present invention stimuli and/or stress means any biotic and abiotic factor which can induce cell death, such as drugs, active ingredients of drugs, environmental effects, environmental contaminants.

Examples of drugs which can induce cell death are Platinum-based antineoplastic drugs, zacitidine, Capecitabine, Carmofur, Cladribine, Clofarabine, Cytarabine, Decitabine, Floxuridine, Fludarabine, Fluorouracil, Gemcitabine, Mercaptopurine, Nelarabine, Pentostatin, Tegafur, Tioguanine, Methotrexate, Pemetrexed, Raltitrexed, Hydroxycarbamide, Irinotecan, Topotecan, Daunorubicin, Doxorubicin, Epirubicin, Idarubicin, Mitoxantrone, Valrubicin, Etoposide, Teniposide, Cabazitaxel, Docetaxel, Paclitaxel, Vinblastine, Vincristine, Vindesine, Vinflunine, Vinorelbine, Bendamustine, Busulfan, Carmustine, Chlorambucil, Chlormethine, Cyclophosphamide, Dacarbazine, Fotemustine, Ifosfamide, Lomustine, Melphalan, Streptozotocin, Temozolomide, Carboplatin, Cisplatin, Nedaplatin, Oxaliplatin, Altretamine, Bleomycin, Bortezomib, Dactinomycin, Estramustine, Ixabepilone, Mitomycin, Procarbazine, Alemtuzumab, Bevacizumab, Cetuximab, Denosumab, Gemtuzumab, ozogamicin, Ibritumomab, tiuxetan, Ipilimumab, Nivolumab, Ofatumumab, Panitumumab, Pembrolizumab, Pertuzumab, Rituximab, Tositumomab, Trastuzumab, Afatinib, Aflibercept, Axitinib, Bosutinib, Crizotinib, Dasatinib, Erlotinib, Gefitinib, Imatinib, Lapatinib, Nilotinib, Pazopanib, Ponatinib, Regorafenib, Ruxolitinib, Sorafenib, Sunitinib, Vandetanib, Everolimus, Temsirolimus, Alitretinoin, Bexarotene, Isotretinoin, Tamibarotene, Tretinoin, Lenalidomide, Pomalidomide, Thalidomide, Panobinostat, Romidepsin, Valproate,

Vorinostatalone or in combination, which are able to induce specific cell death through intrinsic and extrinsic apototosis. For example, Paclitaxel is able to induce pyroptosis on A549 cell line in combination with platin.

Examples of active ingredients of drugs which can induce cell death are cycloastragenol, curcumin, radicicol, resveratrol, which are active ingredients of natural origin which induce cell death activating specific molecular pathways.

Examples of environmental effects and environmental contaminants which can induce cell death are anthropogenic activities, for example metalworking and industrial processes. Said activities contribute to disperse environmental contaminants such as metals (aluminium, arsenic, cadmium, mercury), PAHs (polycyclic aromatic hydrocarbons), POPs (persistent organic pollutants, such as dichlorodiphenyl, trichloroethane, Dithiothreitol, biphenyls, polychlorinated dibenzo-p-dioxins and polychlorinated dibenzofurans).

Within the meaning of the present invention a set of known genes involved in cell death signalling pathway means genes involved in cellular signalling leading to cell death.

Examples of genes involved in cell death signalling pathway, in particular apoptosis, in the pro-apoptotic phase are: ABL1, APAF1, ATP6V1G2, BAX, BCL2L11, BIRC2 (c-IAP1), CASP1 (ICE), CASP3, CASP6, CASP7, CASP9, CD40 (TNFRSF5), CD40LG (TNFSF5), CFLAR (Casper), CYLD, DFFA, FAS (TNFRSF6), FASLG (TNFSF6), GADD45A, NOL3, SPATA2, SYCP2, TNF, TNFRSF1A (TNFR1), TNFRSF10A (TRAIL-R), TP53 (p53).

Examples of genes involved in cell death signalling pathway, in particular autophagy are: AKT1, APP, ATG12, ATG16L1, ATG3, ATG5, ATG7, BAX, BCL2, BCL2L1 (BCLXL), BECN1, CASP3, CTSB, CTSS, ESR1 (ERα), FAS (TNFRSF6), GAA, HTT, IFNG, IGF1, INS, IRGM, MAP1LC3A, MAPK8 (JNK1), NFKB1, PIK3C3 (Vps34), RPS6KB1, SNCA, SQSTM1, TNF, TP53 (p53), ULK1.

Examples of genes involved in cell death signalling pathway, in particular necrosis are: ATP6V1G2, BMF, C1orf159, CCDC103, COMMD4, CYLD, DEFB1, DENND4A, DPYSL4, EIF5B, FOXI1, GALNT5, GRB2, HSPBAP1, JPH3, KCNIP1, MAG, OR10J3, PARP1 (ADPRT1), PARP2, PVR, RAB25, S100A7A, SPATA2, SYCP2, TMEM57, TNFRSF1A (TNFR1), TXNL4B.

Within the meaning of the present invention gene expression analysis means any method for qualification and quantification of expression of at least one gene. Examples of methods for gene expression analysis are: qPCR, Array based technologies, Transcript sequences and abundance detection methods (RNAseq, EST sequencing, Sage).

Within the meaning of the present invention survival means percentage of cell(s) not undergoing to cell death.

Within the meaning of the present invention housekeeping gene means any gene involved in the maintenance of basic cellular functions.

Examples of housekeeping gene are: ACTB gene encoding for Actin beta, B2M gene encoding for Beta-2-microglobulin, GAPDH gene encoding for Glyceraldehyde-3-phosphate dehydrogenase, HPRT1 gene encoding for Hypoxanthine phosphoribosyltransferase 1, HSP90AB1 gene encoding for Heat shock protein 90kDa alpha cytosolic class B member 1, LDHA gene encoding for Lactate dehydrogenase A, NONO gene encoding for Non-POU domain containing octamer-binding, PGK1 gene encoding for Phosphoglycerate kinase 1, PPIH gene encoding for Peptidylprolyl isomerase H (cyclophilin H), RPLP0 gene encoding for Ribosomal protein large P0, TFRC gene encoding for Transferrin receptor (p90, CD71), PGK1 gene encoding for Phosphoglycerate kinase 1, PPIH gene encoding for Peptidylprolyl isomerase H (cyclophilin H), GUSB gene encoding for Glucuronidase beta, TFRC gene encoding for Transferrin receptor (p90, CD71).

With reference to the claims the above technical problem is solved by providing an in vitro or ex vivo method to predict the cell fate and/or death in response to stimuli and/or stresses, comprising the following steps:
a) collecting a cellular sample from biological material
b) selecting a set of known genes (A to M) involved in cell signalling pathways of the cells contained in the cellular sample from biological material of step a)
c) exposing the cellular sample from biological material of step a) to a stressor element
d) subjecting the cellular sample from biological material exposed to a stressor element as obtained in step c) to gene expression analysis to obtain genes belonging to the set of genes as selected in step b)
e) analysing the data regarding gene expression analysis as obtained in step e) by the algorithm 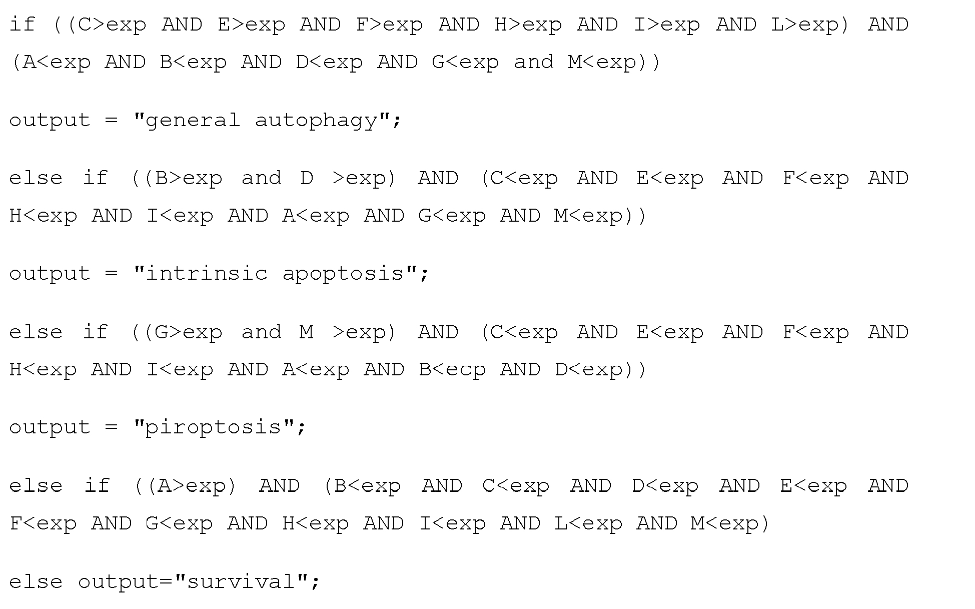
   wherein A to M are genes selected in step b)
   wherein exp is the two fold value of the expression level calculated with the equation: exp=2^((-average(Delta ct (gene expression in treated cells)))/(-average(Delta ct gene expression in untreated cells)))
   wherein Delta ct= Ct value reference gene - average of the Ct values of at least 3 housekeeping genes
   to calculate the scores of outputs: general autophagy, intrinsic apoptosis, piroptosis, survival
   wherein the output with the higher value is predictive of the cell fate.

The cellular sample from biological material of step a) is a biological sample containing cells whose genome comprises genes involved in cellular signalling leading to cell death and housekeeping genes.

Preferably the cellular sample from biological material can be selected from the group consisting of tissue sample, primary cell cultures, secondary cell cultures, cell lines from collection, blood sample.

Preferably the cellular sample from biological material is of human origin.

Preferably cells and/or tissues can be normal or cancerous depending on the study to be conducted.

The cellular sample from biological material can be in vitro processed, for example cell can be cultured by using techniques well known in the common general knowledge. Culture medium, supplementation and culturing conditions depend on the cell line used.

Preferably the set of genes selected in the step b) comprises at least one gene of the group consisting of: ABL1, APAF1, ATP6V1G2, BAX, BCL2L11, BIRC2 (c-IAP1), CASP1 (ICE), CASP3, CASP6, CASP7, CASP9, CD40 (TNFRSF5), CD40LG (TNFSF5), CFLAR (Casper), CYLD, DFFA, FAS (TNFRSF6), FASLG (TNFSF6), GADD45A, NOL3, SPATA2, SYCP2, TNF, TNFRSF1A (TNFR1), TNFRSF10A (TRAIL-R), TP53 (p53) AKT1, APP, ATG12, ATG16L1, ATG3, ATG5, ATG7, BAX, BCL2, BCL2L1 (BCLXL), BECN1, CASP3, CTSB, CTSS, ESR1 (ER*α*), FAS (TNFRSF6), GAA, HTT, IFNG, IGF1, INS, IRGM, MAP1LC3A, MAPK8 (JNK1), NFKB1, PIK3C3 (Vps34), RPS6KB1, SNCA, SQSTM1, TNF, TP53 (p53), ULK1, ATP6V1G2, BMF, C1orf159, CCDC103, COMMD4, CYLD, DEFB1, DENND4A, DPYSL4, EIF5B, FOXI1, GALNT5, GRB2, HSPBAP1, JPH3, KCNIP1, MAG, OR10J3, PARP1 (ADPRT1), PARP2, PVR, RAB25, S100A7A, SPATA2, SYCP2, TMEM57, TNFRSF1A (TNFR1), TXNL4B; beta B2M, GAPDH, HPRT1, HSP90AB1, LDHA, NONO, PGK1, PPIH, RPLP0, TFRC, PGK1, PPIH, GUSB, TFRC.

More preferably the set of genes selected in step b), involved in cell signalling pathways, comprises the genes as defined in the following table 1

**Table 1**

| CODE | GENE CONVENTIONAL NAME | ACTUAL ACCESSION NUMBER OF THE GENE TRANSCRIPT AT NCBI |
|---|---|---|
| A | TNF | NM_000594 |
| B | CASP7 | NM_001227 |
| C | SQSTM1 | NM_003900 |
| D | CASP3 | NM_004346 |
| E | ATG12 | NM_004707 |
| F | ATG5 | NM_004849 |
| G | DEFB1 | NM_005218 |
| H | ATG7 | NM_006395 |
| I | ATG16L1 | NM_017974 |
| L | ATG3 | NM_0022488 |
| M | CASP1 | NM_033292 |

Preferably in step c) stimuli and/or stresses are selected from the group consisting of: Platinum-based antineoplastic drugs, zacitidine, Capecitabine, Carmofur, Cladribine, Clofarabine, Cytarabine, Decitabine, Floxuridine, Fludarabine, Fluorouracil, Gemcitabine, Mercaptopurine, Nelarabine, Pentostatin, Tegafur, Tioguanine, Methotrexate, Pemetrexed, Raltitrexed, Hydroxycarbamide, Irinotecan, Topotecan, Daunorubicin, Doxorubicin, Epirubicin, Idarubicin, Mitoxantrone, Valrubicin, Etoposide, Teniposide, Cabazitaxel, Docetaxel, Paclitaxel, Vinblastine, Vincristine, Vindesine, Vinflunine, Vinorelbine, Bendamustine, Busulfan, Carmustine, Chlorambucil, Chlormethine, Cyclophosphamide, Dacarbazine, Fotemustine, Ifosfamide, Lomustine, Melphalan, Streptozotocin, Temozolomide, Carboplatin, Cisplatin, Nedaplatin, Oxaliplatin, Altretamine, Bleomycin, Bortezomib, Dactinomycin, Estramustine, Ixabepilone, Mitomycin, Procarbazine, Alemtuzumab, Bevacizumab, Cetuximab, Denosumab, Gemtuzumab, ozogamicin, Ibritumomab, tiuxetan, Ipilimumab, Nivolumab, Ofatumumab, Panitumumab, Pembrolizumab, Pertuzumab, Rituximab, Tositumomab, Trastuzumab, Afatinib, Aflibercept, Axitinib, Bosutinib, Crizotinib, Dasatinib, Erlotinib, Gefitinib, Imatinib, Lapatinib, Nilotinib, Pazopanib, Ponatinib, Regorafenib, Ruxolitinib, Sorafenib, Sunitinib, Vandetanib, Everolimus, Temsirolimus, Alitretinoin, Bexarotene, Isotretinoin, Tamibarotene, Tretinoin, Lenalidomide, Pomalidomide, Thalidomide, Panobinostat, Romidepsin, Valproate, Vorinostat and combination thereof; aluminium, arsenic, cadmium, mercury, polycyclic aromatic hydrocarbons, persistent organic pollutants, dichlorodiphenyl, trichloroethane, Dithiothreitol, biphenyls, polychlorinated dibenzo-p-dioxins and polychlorinated dibenzofurans and combination thereof.

Preferably in step d) gene expression analysis is conducted by a method selected from the group consisting of: qPCR, Array based technologies, RNAseq, EST sequencing, Sage.

The method object of the present invention, can be used for research purposes, such as screening and drug discovery.

The method object of the present invention, can be used for clinical purposes, such as anticancer therapy decision-making, in which an important aspect is the early identification of the specific cellular signalling pathway targeted by the bioactive molecule in order to predict the type of cancer that can be treated with a drug; or to predict how a patient may respond to treatment thereby avoiding side effects and selecting the appropriate drug therapy; or to analyse the effects of the drug therapy thereby adapting the therapy at an early stage of the disease.

### Examples

The treatment of lung adenocarcinoma cell line (A549) with a pure chemical fraction obtained from the marine microalga *Cyclotella cryptica.*

Cell lines were usually purchased from the American Type Culture Collection (ATCC^{®} CCL185^{™}) and grown in specific culture media supplemented with 10% (v/v) of fetal bovine serum (FBS), 100 units ml-1 penicillin and 100 µg ml-1 streptomycin and other essential nutrients such as amino acids (depending on cell line cultivated). The medium was renewed every 3 days, and the cells were detached via trypsinization when they reached confluence. Before the experiments, cells were seeded in Petri dishes (100 mm diameter, 2 × 106 cells well-1) and kept overnight for attachment.

After the exposure time, cells were washed directly in the Petri dish by adding cold Phosphate-Buffered Saline (PBS) and shaking gently.

Cells were lysed in the Petri dish by adding lysis reagent. RNA was isolated according to the standard protocol.

About 200 ng RNA was subjected to reverse transcription reaction.

The term "pyroptosis" was originally introduced to describe a particular form of cell death in macrophages, which is induced by bacterial infection, is accompanied by caspase-1 activation and hence leads to the release of pyrogenic interleukins; however, it is still controversial whether pyroptosis - which can also be triggered by nonbacterial pathological stimuli - truly represents a cell death modality on its own or whether it constitutes a special case of apoptosis or necrosis. Pyroptosis is morphologically and mechanistically distinct from other forms of cell death. Caspase 1 dependence is a defining feature of pyroptosis, and caspase 1 is the enzyme that mediates this process of cell death

### Genes upregulated

BAX (BCL2-Associated X Protein)=This protein forms a heterodimer with BCL2, and functions as an apoptotic activator.

CASP1(Caspase-1)=This gene was identified by its ability to proteolytically cleave and activate the inactive precursor of interleukin-1, a cytokine involved in processes such as inflammation, septic shock, pyroptosis and wound healing.

IL1A (Interleukin 1 alpha)=The protein encoded by this gene is a member of the interleukin 1 cytokine family. This cytokine is a pleiotropic cytokine involved in various immune responses, inflammatory processes, and hematopoiesis.

CYP7A1(Cytochrome P450 7A1)=The cytochrome P450 proteins are monooxygenases which catalyze many reactions involved in drug metabolism and synthesis of cholesterol, steroids and other lipids.

ERCC1 (Excision Repair Cross-Complementation Group 1)= The product of this gene functions in the nucleotide excision repair pathway.

HSP90AA2(Heat Shock 90kDa Protein 1)=HSP90 proteins are highly conserved molecular chaperones that have key roles in signal transduction, protein folding, protein degradation, and morphologic evolution.

HSPD1(Heat Shock 60kDa Protein 1)=The encoded mitochondrial protein may function as a signalling molecule in the innate immune system.

Results are shown in figure 1.

The Experimental results confirmed, at a molecular and a morphological level, that pyroptosis occurred.

No genes implicated in other cell death processes, such as necrosis, autophagy and apoptosis are differently regulated in the cells treated with Cyclotella (fraction F) compared to the control.

The downregulation of the Bax gene further excluded apoptosis.

In particular, pyroptosis was confirmed by the up-regulation of the caspase 1 and interleukin 1a (or alpha) gene.

Moreover, the upregulation of ERCC1 gene, involved in DNA fragmentation repair, confirmed that the process was activated after the involvement of casapase 1.

At a morphological level, the cells presented vesicles ready for secretion and evident poration of the plasma membrane.

## Claims

1. In vitro or ex vivo method to predict the cell fate and/or death in response to stimuli and/or stresses, comprising the following steps:
a) collecting a cellular sample from biological material
b) selecting a set of known genes A to M involved in cell signalling pathways of the cells contained in the cellular sample from biological material of step a)
c) exposing the cellular sample from biological material of step a) to a stressor element
d) subjecting the cellular sample from biological material exposed to a stressor element as obtained in step c) to gene expression analysis to obtain genes belonging to the set of genes as selected in step b)
e) analysing the data regarding gene expression analysis as obtained in step e) by the algorithm 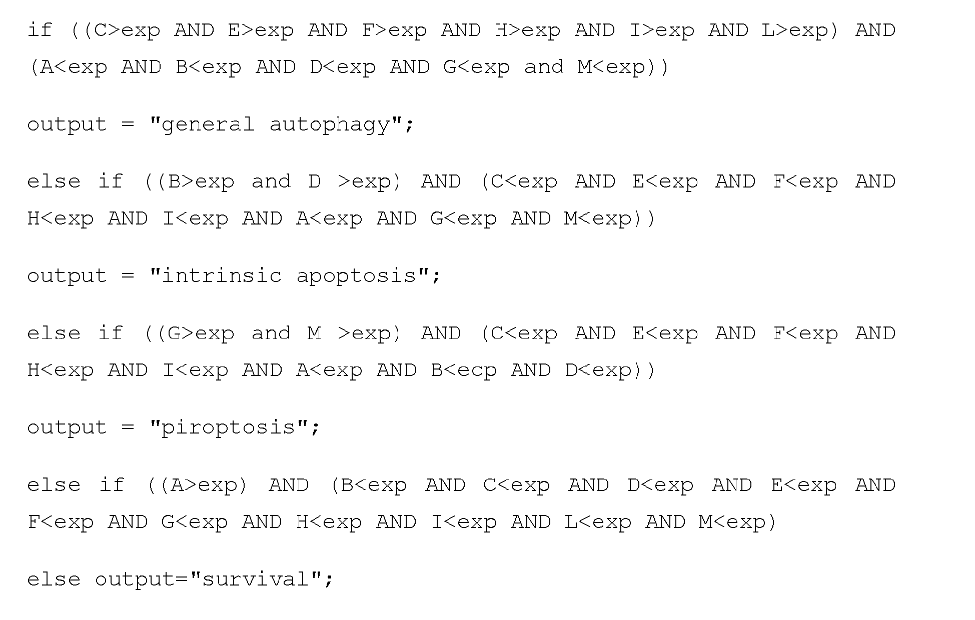
wherein A to M are genes selected in step b)
wherein exp is the two fold value of the expression level calculated with the equation: exp=2^((-average(Delta ct (gene expression in treated cells)))/(-average(Delta ct gene expression in untreated cells)))
wherein Delta ct= Ct value reference gene - average of the Ct values of at least 3 housekeeping genes
to calculate the scores of outputs: general autophagy, intrinsic apoptosis, piroptosis, survival
wherein the output with the higher value is predictive of the cell fate
wherein the set of genes A to M is
| CODE | GENE CONVENTIONAL NAME | ACTUAL ACCESSION NUMBER OF THE GENE TRANSCRIPT AT NCBI |
|---|---|---|
| A | TNF | NM_000594 |
| B | CASP7 | NM_001227 |
| C | SQSTM1 | NM_003900 |
| D | CASP3 | NM_004346 |
| E | ATG12 | NM_004707 |
| F | ATG5 | NM_004849 |
| G | DEFB1 | NM_005218 |
| H | ATG7 | NM_006395 |
| I | ATG16L1 | NM_017974 |
| L | ATG3 | NM_0022488 |
| M | CASP1 | NM_033292 |

2. In vitro or ex vivo method to predict the cell fate and/or death in response to stimuli and/or stresses according to claim 1 wherein the cellular sample from biological material of step a) is a biological sample containing cells whose genome comprises genes involved in cellular signalling leading to cell death and housekeeping genes.

3. In vitro or ex vivo method to predict the cell fate and/or death in response to stimuli and/or stresses according to claim 1 wherein the cellular sample from biological material of step a) is of human origin.

4. In vitro or ex vivo method to predict the cell fate and/or death in response to stimuli and/or stresses according to claim 2 wherein the cellular sample from biological material is selected from the group consisting of tissue sample, primary cell cultures, secondary cell cultures, cell lines from collection, blood sample.

5. In vitro or ex vivo method to predict the cell fate and/or death in response to stimuli and/or stresses according to claim 4 wherein cells and/or tissues are normal or cancerous.

6. In vitro or ex vivo method to predict the cell fate and/or death in response to stimuli and/or stresses according to claim 1 wherein in step c) stimuli and/or stresses are selected from the group consisting of: Platinum-based antineoplastic drugs, zacitidine, Capecitabine, Carmofur, Cladribine, Clofarabine, Cytarabine, Decitabine, Floxuridine, Fludarabine, Fluorouracil, Gemcitabine, Mercaptopurine, Nelarabine, Pentostatin, Tegafur, Tioguanine, Methotrexate, Pemetrexed, Raltitrexed, Hydroxycarbamide, Irinotecan, Topotecan, Daunorubicin, Doxorubicin, Epirubicin, Idarubicin, Mitoxantrone, Valrubicin, Etoposide, Teniposide, Cabazitaxel, Docetaxel, Paclitaxel, Vinblastine, Vincristine, Vindesine, Vinflunine, Vinorelbine, Bendamustine, Busulfan, Carmustine, Chlorambucil, Chlormethine, Cyclophosphamide, Dacarbazine, Fotemustine, Ifosfamide, Lomustine, Melphalan, Streptozotocin, Temozolomide, Carboplatin, Cisplatin, Nedaplatin, Oxaliplatin, Altretamine, Bleomycin, Bortezomib, Dactinomycin, Estramustine, Ixabepilone, Mitomycin, Procarbazine, Alemtuzumab, Bevacizumab, Cetuximab, Denosumab, Gemtuzumab, ozogamicin, Ibritumomab, tiuxetan, Ipilimumab, Nivolumab, Ofatumumab, Panitumumab, Pembrolizumab, Pertuzumab, Rituximab, Tositumomab, Trastuzumab, Afatinib, Aflibercept, Axitinib, Bosutinib, Crizotinib, Dasatinib, Erlotinib, Gefitinib, Imatinib, Lapatinib, Nilotinib, Pazopanib, Ponatinib, Regorafenib, Ruxolitinib, Sorafenib, Sunitinib, Vandetanib, Everolimus, Temsirolimus, Alitretinoin, Bexarotene, Isotretinoin, Tamibarotene, Tretinoin, Lenalidomide, Pomalidomide, Thalidomide, Panobinostat, Romidepsin, Valproate, Vorinostat and combination thereof; aluminium, arsenic, cadmium, mercury, polycyclic aromatic hydrocarbons, persistent organic pollutants, dichlorodiphenyl, trichloroethane, Dithiothreitol, biphenyls, polychlorinated dibenzo-p-dioxins and polychlorinated dibenzofurans and combination thereof.

7. In vitro or ex vivo method to predict the cell fate and/or death in response to stimuli and/or stresses according to claim 1 wherein in step d) gene expression analysis is conducted by a method selected from the group consisting of: qPCR, Array based technologies, RNAseq, EST sequencing, Sage.

## Patentansprüche

1. In-vitro- oder Ex-vivo-Verfahren zur Vorhersage des Zellschicksals und/oder des Zelltodes als Reaktion auf Reize und/oder Stress, umfassend die folgenden Schritte:
a) Entnahme einer Zellprobe aus biologischem Material
b) Auswählen eines Satzes bekannter Gene A bis M, die an Zellsignalwegen der in der Zellprobe enthaltenen Zellen aus biologischem Material von Schritt a) beteiligt sind
c) Exponieren der Zellprobe aus biologischem Material aus Schritt a) zu einem Stressfaktorelement
d) Unterziehen der Zellprobe aus biologischem Material, das einem Stressfaktorelement ausgesetzt ist, wie in Schritt c) erhalten, einer Analyse, um Gene zu erhalten, die zu den in Schritt b) ausgewählten Genen gehören, einer Genexpressionsanalyse, um Gene zu erhalten, die zu dem in Schritt b) ausgewählten Satz von Genen gehören
e) Analysieren der in Schritt e) erhaltenen Daten zur Genexpressionsanalyse durch den Algorithmus
```
     wenn ((C>exp UND E>exp UND F>exp UND H>exp UND I>exp UND
     L>exp) UND (A<exp UND B<exp UND D<exp UND G<exp UND M<exp))
     Ausgabe = "allgemeine Autophagie";
     sonst wenn ((B>exp UND D >exp) UND (C<exp UND E<exp UND F<exp
     UND H<exp UND I<exp UND A<exp UND G<exp UND M<exp))
     Ausgabe = "intrinsische Apoptose";
     sonst wenn ((G>exp UND M >exp) UND (C<exp UND E<exp UND F<exp
     UND H<exp UND I<exp UND A<exp UND B<ecp UND D<exp))
     Ausgabe = "Pyroptose";
     sonst wenn ((A>exp) UND (B<exp UND C<exp UND D<exp UND E<exp
     UND F<exp UND G<exp UND H<exp UND I<exp UND L<exp UND
     M<exp))
     sonst Ausgabe = "Überleben";
```
wobei A bis M Gene sind, die in Schritt b) ausgewählt wurden.
wobei exp der zweifache Wert des Expressionsniveaus ist, berechnet mit der Gleichung: exp=2^((-Mittelwert (Delta ct (Genexpression in behandelten Zellen)))/(- Mittelwert (Delta ct-Genexpression in unbehandelten Zellen)))
wobei Delta ct = CT-Wert-Referenzgen - Durchschnitt der CT-Werte von mindestens 3 Haushaltsgenen
um die Ergebnisse der Ausgaben zu berechnen: allgemeine Autophagie, intrinsische Apoptose, Pyroptose, Überleben
wobei die Ausgabe mit dem höheren Wert das Schicksal der Zelle vorhersagt
wobei der Satz der Gene A bis M ist
| CODE | KONVENTIONALER GENNAME | TATSACHLICHE ZUGRIFFSNUMMER DES GENTRANSKRIPTS BEI NCBI |
|---|---|---|
| A | TNF | NM_000594 |
| B | CASP7 | NM_001227 |
| C | SQSTM1 | NM_003900 |
| D | CASP3 | NM_004346 |
| E | ATG12 | NM_004707 |
| F | ATGS | NM_004849 |
| G | DEFB1 | NM_005218 |
| H | ATG7 | NM_006395 |
| I | ATG16L1 | NM_017974 |
| L | ATG3 | NM_0022488 |
| M | CASP1 | NM_033292 |

2. In-vitro- oder Ex-vivo-Verfahren zur Vorhersage des Zellschicksals und/oder des Zelltodes als Reaktion auf Reize und/oder Stress gemäß Anspruch 1, wobei die Zellprobe aus biologischem Material aus Schritt a) eine biologische Probe ist, welche Zellen enthält, deren Genom Gene umfasst, die an der zellulären Signalübertragung beteiligt sind, die zum Zelltod führt, sowie Haushaltsgene.

3. In-vitro- oder Ex-vivo-Verfahren zur Vorhersage des Zellschicksals und/oder des Zelltodes als Reaktion auf Reize und/oder Stress gemäß Anspruch 1, wobei die Zellprobe aus biologischem Material aus Schritt a) menschlichen Ursprungs ist.

4. In-vitro- oder Ex-vivo-Verfahren zur Vorhersage des Zellschicksals und/oder des Zelltodes als Reaktion auf Reize und/oder Stress gemäß Anspruch 2, wobei die Zellprobe aus biologischem Material aus gewählt ist aus der Gruppe bestehend aus Gewebeproben, primäre Zellkulturen, sekundäre Zellkulturen, Zelllinien aus der Sammlung, Blutproben.

5. In-vitro- oder Ex-vivo-Verfahren zur Vorhersage des Zellschicksals und/oder des Zelltodes als Reaktion auf Reize und/oder Stress gemäß Anspruch 4, wobei wobei Zellen und/oder Gewebe normal oder krebsartig sind.

6. In-vitro- oder Ex-vivo-Verfahren zur Vorhersage des Zellschicksals und/oder des Zelltodes als Reaktion auf Reize und/oder Stress gemäß Anspruch 1, wobei in Schritt c) Reize und/oder Stresse ausgewählt sind aus der Gruppe bestehend aus: Antineoplastische Medikamente auf Platinbasis, Azacitidin, Capecitabin, Carmofur, Cladribin, Clofarabin, Cytarabin, Decitabin, Floxuridin, Fludarabin, Fluorouracil, Gemcitabin, Mercaptopurin, Nelarabin, Pentostatin, Tegafur, Tioguanin, Methotrexat, Pemetrexed, Raltitrexed, Hydroxycarbamid, Irinotecan, Topotecan, Daunorubicin, Doxorubicin, Epirubicin, Idarubicin, Mitoxantron, Valrubicin, Etoposid, Teniposid, Cabazitaxel, Docetaxel, Paclitaxel, Vinblastin, Vincristin, Vindesin, Vinflunin, Vinorelbin, Bendamustin, Busulfan, Carmustin, Chlorambucil, Chlormethin, Cyclophosphamid, Dacarbazin, Fotemustin, Ifosfamid, Lomustin, Melphalan, Streptozotocin, Temozolomid, Carboplatin, Cisplatin, Nedaplatin, Oxaliplatin, Altretamin, Bleomycin, Bortezomib, Dactinomycin, Estramustin, Ixabepilon, Mitomycin, Procarbazin, Alemtuzumab, Bevacizumab, Cetuximab, Denosumab, Gemtuzumab, Ozogamicin, Ibritumomab, Tiuxetan, Ipilimumab, Nivolumab, Ofatumumab, Panitumumab, Pembrolizumab, Pertuzumab, Rituximab, Tositumomab, Trastuzumab, Afatinib, Aflibercept, Axitinib, Bosutinib, Crizotinib, Dasatinib, Erlotinib, Gefitinib, Imatinib, Lapatinib, Nilotinib, Pazopanib, Ponatinib, Regorafenib, Ruxolitinib, Sorafenib, Sunitinib, Vandetanib, Everolimus, Temsirolimus, Alitretinoin, Bexaroten, Isotretinoin, Tamibaroten, Tretinoin, Lenalidomid, Pomalidomid, Thalidomid, Panobinostat, Romidepsin, Valproat, Vorinostat und Kombinationen davon; Aluminium, Arsen, Cadmium, Quecksilber, polyzyklische aromatische Kohlenwasserstoffe, persistente organische Schadstoffe, Dichlordiphenyl, Trichlorethan, Dithiothreitol, Biphenyle, polychlorierte Dibenzo-p-dioxine und polychlorierte Dibenzofurane und Kombinationen davon.

7. In-vitro- oder Ex-vivo-Verfahren zur Vorhersage des Zellschicksals und/oder des Zelltodes als Reaktion auf Reize und/oder Stress gemäß Anspruch 1, wobei in Schritt d) die Genexpressionsanalyse mit einer Methode durchgeführt wird, die aus der Gruppe ausgewählt ist, bestehend aus: qPCR, Array-basierten Technologien, RNAseq, EST-Sequenzierung, Sage.

## Revendications

1. Procédé in vitro ou ex vivo pour prédire le destin et/ou la mort cellulaire en réponse à des stimuli et/ou des stress, comprenant les étapes suivantes:
a) prélèvement d'un échantillon cellulaire de matériel biologique
b) sélection d'un ensemble de gènes A à M connus impliqués dans des voies de signalisation cellulaire des cellules contenues dans l'échantillon cellulaire de matériel biologique de l'étape a)
c) exposition de l'échantillon cellulaire de matériel biologique de l'étape a) à un élément stressant
d) soumission de l'échantillon cellulaire de matériel biologique exposé à un élément stressant comme obtenu dans l'étape c) à une analyse d'expression génique pour obtenir des gènes appartenant à l'ensemble de gènes sélectionné dans l'étape b)
e) analyse des données concernant l'analyse d'expression génique comme obtenu dans l'étape e) par l'algorithme
```
   si ((C>exp AND E>exp AND F>exp AND H>exp AND I>exp AND L>exp)
   AND (A<exp AND B<exp AND D<exp AND G<exp AND M<exp))
   sortie = "autophagie générale";
   sinon si ((B>exp and D >exp) AND (C<exp AND E<exp AND F<exp AND
   H<exp AND I<exp AND A<exp AND G<exp AND M<exp))
   sortie = "apoptose intrinsèque";
   sinon si ((G>exp and M >exp) AND (C<exp AND E<exp AND F<exp AND
   H<exp AND I<exp AND A<exp AND B<ecp AND D<exp))
   sortie = "piroptose";
   sinon si ((A>exp) AND (B<exp AND C<exp AND D<exp AND E<exp AND
   F<exp AND G<exp AND H<exp AND I<exp AND L<exp AND M<exp)
   sinon sortie = "survie";
```
où A à M sont des gènes sélectionnés dans l'étape b)
où exp est la valeur multipliée par deux du niveau d'expression calculé avec l'équation: exp=2^ ((-moyenne (Delta et (expression génique dans les cellules traitées)))/(-moyenne (Delta et expression génique dans les cellules non traitées))) où Delta et = valeur Ct gène de référence - moyenne des valeurs Ct d'au moins 3 gènes domestiques
calculer les scores de sorties: autophagie générale, apoptose intrinsèque, piroptose, survie
où la sortie avec la valeur la plus élevée est prédictive du destin cellulaire
où l'ensemble de gènes A à M est
| CODE | NOM CONVENTIONNEL DU GENE | NUMERO D' ORDRE ACTUEL DU TRANSCRIT DE GENE A NCBI |
|---|---|---|
| A | TNF | NM_000594 |
| B | CASP7 | NM_001227 |
| C | SQSTM1 | NM_003900 |
| D | CASP3 | NM_004346 |
| E | ATG12 | NM_004707 |
| F | ATG5 | NM_004849 |
| G | DEFB1 | NM_005218 |
| H | ATG7 | NM_006395 |
| I | ATG16L1 | NM_017974 |
| L | ATG3 | NM_0022488 |
| M | CASP1 | NM_033292 |

2. Procédé in vitro ou ex vivo pour prédire le destin et/ou la mort cellulaire en réponse à des stimuli et/ou des stress selon la revendication 1 où l'échantillon cellulaire de matériel biologique de l'étape a) est un échantillon biologique contenant des cellules dont le génome comprend des gènes impliqués dans la signalisation cellulaire conduisant à la mort cellulaire et des gènes domestiques.

3. Procédé in vitro ou ex vivo pour prédire le destin et/ou la mort cellulaire en réponse à des stimuli et/ou des stress selon la revendication 1 où l'échantillon cellulaire de matériel biologique de l'étape a) est d'origine humaine.

4. Procédé in vitro ou ex vivo pour prédire le destin et/ou la mort cellulaire en réponse à des stimuli et/ou des stress selon la revendication 2 où l'échantillon cellulaire de matériel biologique est choisi dans le groupe consistant en un échantillon de tissu, des cultures cellulaires primaires, des cultures cellulaires secondaires, des lignées cellulaires de prélèvement, un échantillon sanguin.

5. Procédé in vitro ou ex vivo pour prédire le destin et/ou la mort cellulaire en réponse à des stimuli et/ou des stress selon la revendication 4 où les cellules et/ou les tissus sont normaux ou cancéreux.

6. Procédé in vitro ou ex vivo pour prédire le destin et/ou la mort cellulaire en réponse à des stimuli et/ou des stress selon la revendication 1 où dans l'étape c) les stimuli et/ou les stress sont choisis dans le groupe consistant en: médicaments antinéoplasiques à base de platine, azacitidine, capécitabine, carmofur, cladribine, clofarabine, cytarabine, décitabine, floxuridine, fludarabine, fluorouracile, gemcitabine, mercaptopurine, nélarabine, pentostatine, tégafur, tioguanine, méthotrexate, pemetrexed, raltitrexed, hydroxycarbamide, irinotécan, topotécan, daunorubicine, doxorubicine, épirubicine, idarubicine, mitoxantrone, valrubicine, étoposide, téniposide, cabazitaxel, docétaxel, paclitaxel, vinblastine, vincristine, vindésine, vinflunine, vinorelbine, bendamustine, busulfan, carmustine, chlorambucil, chlorméthine, cyclophosphamide, dacarbazine, fotemustine, ifosfamide, lomustine, melphalan, streptozotocine, témozolomide, carboplatine, cisplatine, nédaplatine, oxaliplatine, altretamine, bléomycine, bortezomib, dactinomycine, estramustine, ixabepilone, mitomycine, procarbazine, alemtuzumab, bevacizumab, cetuximab, denosumab, gemtuzumab, ozogamicine, ibritumomab, tiuxetan, ipilimumab, nivolumab, ofatumumab, panitumumab, pembrolizumab, pertuzumab, rituximab, tositumomab, trastuzumab, afatinib, aflibercept, axitinib, bosutinib, crizotinib, dasatinib, erlotinib, gefitinib, imatinib, lapatinib, nilotinib, pazopanib, ponatinib, regorafenib, ruxolitinib, sorafenib, sunitinib, vandetanib, everolimus, temsirolimus, alitrétinoïne, bexarotène, isotrétinoïne, tamibarotène, trétinoïne, lénalidomide, pomalidomide, thalidomide, panobinostat, romidepsine, valproate, vorinostat et une combinaison de ceux-ci; aluminium, arsenic, cadmium, mercure, hydrocarbures aromatiques polycycliques, polluants organiques persistants, dichlorodiphényle, trichloroéthane, dithiothréitol, biphényles, dibenzo-p-dioxines polychlorées et dibenzofuranes polychlorés et une combinaison de ceux-ci.

7. Procédé in vitro ou ex vivo pour prédire le destin et/ou la mort cellulaire en réponse à des stimuli et/ou des stress selon la revendication 1 où dans l'étape d) l'analyse de l'expression génique est effectuée par une procédé choisi dans le groupe consistant en: qPCR, les technologies basées sur des puces, RNAseq, séquençage EST, Sage.
